# EUROPEAN PATENT SPECIFICATION

(11) **EP 1 937 194 B1**
(45) Date of publication and mention of the grant of the patent: **18.11.2009**
(21) Application number: 07803378.4
(22) Date of filing: 10.09.2007
(51) Int. Cl.: A61F 5/02

(54) **LUMBAR BACK BRACE SUPPORT MEMBER FOR A BACK BRACE SUPPORT BELT**
LUMBARRÜCKSCHIENENSTÜTZGLIED FÜR EINEN RÜCKENSTÜTZGURT
ÉLÉMENT DE SUPPORT POUR CEINTURE LOMBAIRE

(30) Priority: 25.09.2006 IT PN20060071
(43) Date of publication of application: 02.07.2008
(73) Proprietor: ABE Sport Group S.r.L., 31041 Cornuda (TV) (IT)
(72) Inventor: BERLESE, Remigio, 31044 Montebelluna (TV) (IT)
(74) Representative: Gonella, Mario
(86) International application number: PCT/EP2007/059461
(87) International publication number: WO 2008/037584

(56) References cited:
- DE-A1- 1 806 685
- US-A- 3 605 731

## Description

The present invention refers to a lumbar back brace support member for a back brace support belt. The invention is particularly, although not solely, suitable for application in the field of support, guarding and protective accessories for motorcyclists, as well as in the medical field as an aid to patient suffering from pains in the back or, anyway, needing adequate support of the spine and the back in general.

Currently available back brace support belts are generally known to feature a rear portion, adapted to wrap around the lumbar region of the wearer's back, which is higher than the front portion to the purpose of ensuring the wearer with the desired back support effect, as well as an adequate protective effect in case of use in the motorcycling field.

US 3,605,731 discloses a device for support, correction and treatment of the human spinal column which includes, as an abdominal and back supporting member, an elastic band with ends respectively adjustably secured to disconnectable halves of a shield-like abdominal plate and, on the inside of a central back region defined between two sewn-in, flexible vertical stiffening ribs, an array of elongated body contact support elements pivotally carried and extending transversely to a vertical spring strip centrally secured on the back region.

A drawback that is connected with these prior-art solutions lies in the fact that, in view of ensuring an adequate support and protection, the rear portion of the belt should be made so as to feature sufficient rigidity. Such rigidity would however prevent the belt from being able to adequately adapt to the movements of the wearer in the various phases of use, thereby limiting the movement abilities of the wearer to a considerable extent. It is therefore necessary to make the support portion of the belt with materials that are sufficiently flexible and capable to adapt to the wearer's movements. As a result, currently existing back brace support belts are a compromise between these two functional requirements: on the one hand, the need for an adequate support and protection effect to be ensured and, on the other hand, the need for the wearer to be ensured a sufficient freedom of movement; in any case, none of these two functions are performed to an optimum extent.

It is therefore an object of the present invention to overcome the drawbacks of known back brace support belts by providing a lumbar back brace support member for a back brace support belt that is effective in both supporting and protecting the wearer's back in an optimum manner, while ensuring him/her with a fully adequate freedom of movement.

Within this general object, it is an important purpose of the present invention to provide a lumbar back brace support member for a back brace support belt that is fully safe and reliable in use, in particular when used in the motorcycling field where the need arises for the wearer to be provided with both a back support effect during riding and a back protection effect in the case of accidental falls.

It is a further purpose of the present invention to provide a lumbar back brace support member for a back brace support belt that is able to provide the wearer with an optimum support effect of all lumbar areas concerned, i.e. both the central area corresponding to the spine region and the side areas corresponding to the renal regions.

Yet another purpose of the present invention is to provide a lumbar back brace support member for a back brace support belt that is capable of being manufactured at fully competitive costs using generally and largely available equipment, materials and technique.

According to the present invention, these aims, along with further ones that will become apparent from the following disclosure, are reached in a lumbar back brace support member for a back brace support belt as defined in claim 1.

Further features and aspects of the present invention are defined in the appended sub-claims.

Features and advantages of the present invention will anyway be readily understood from the description of a preferred, although not sole embodiment that is given below by way of non-limiting example with reference to the accompanying drawings, in which:

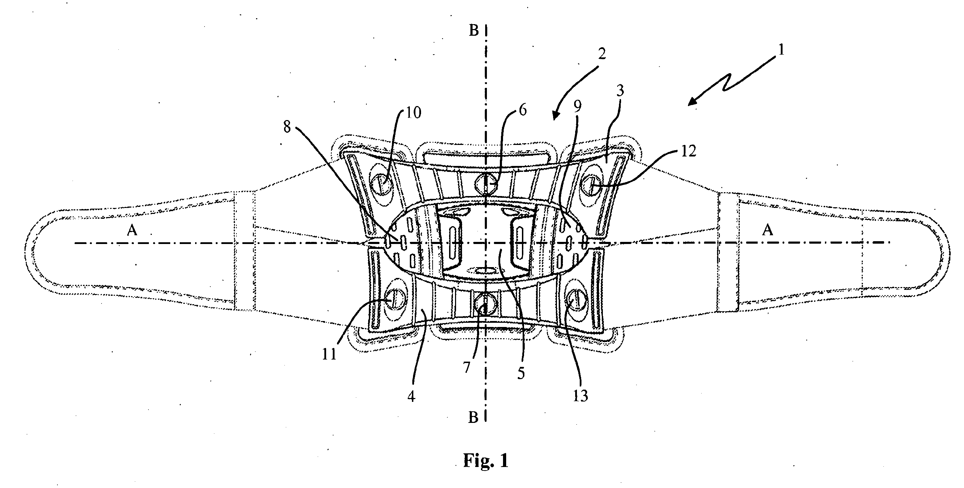

- Figure 1 shows a back brace support belt comprising a lumbar back brace support member according to the present invention;

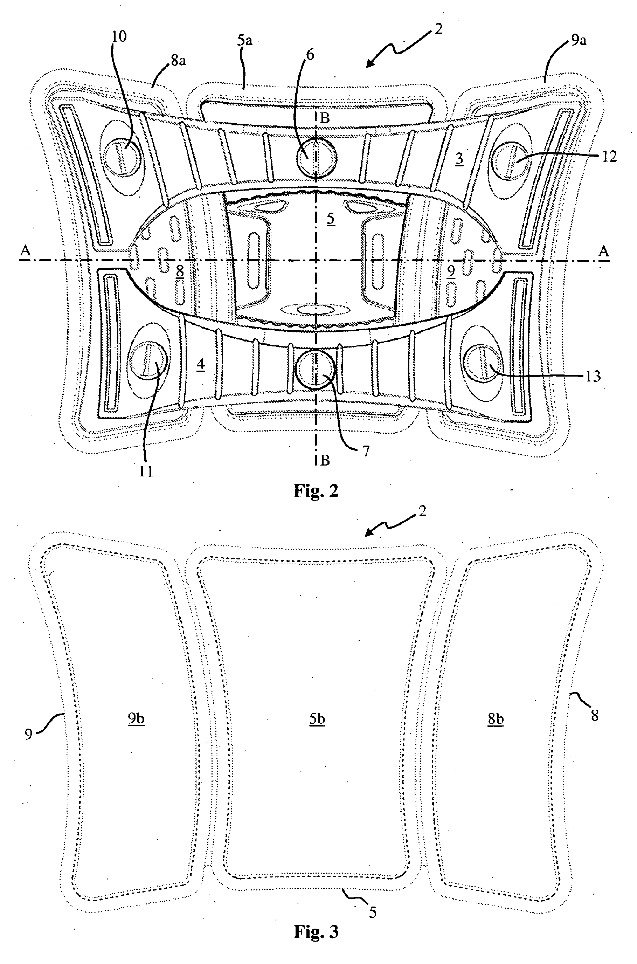

- Figure 2 is a front elevational view of a lumbar back brace support member according to the present invention, as viewed from the side opposite to the one intended for resting against the wearer's back;
- Figure 3 is a front elevational view of a lumbar back brace support member shown in Figure 2, as viewed from the side intended for resting against the wearer's back;

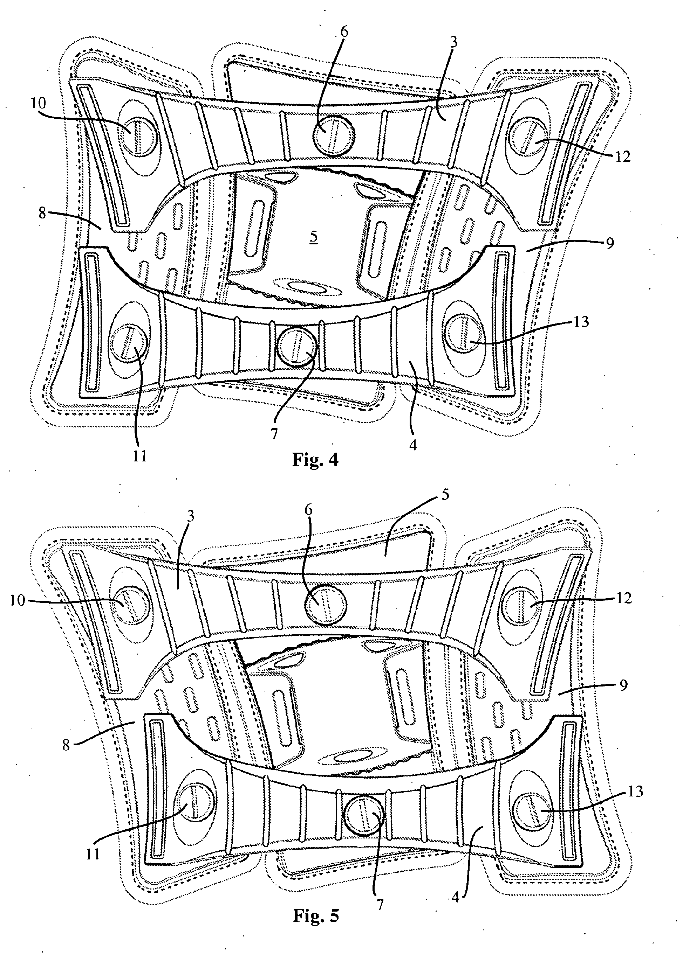

- Figures 4 and 5 are illustrative of the possibilities of movement of the lumbar back brace support member according to the present invention;

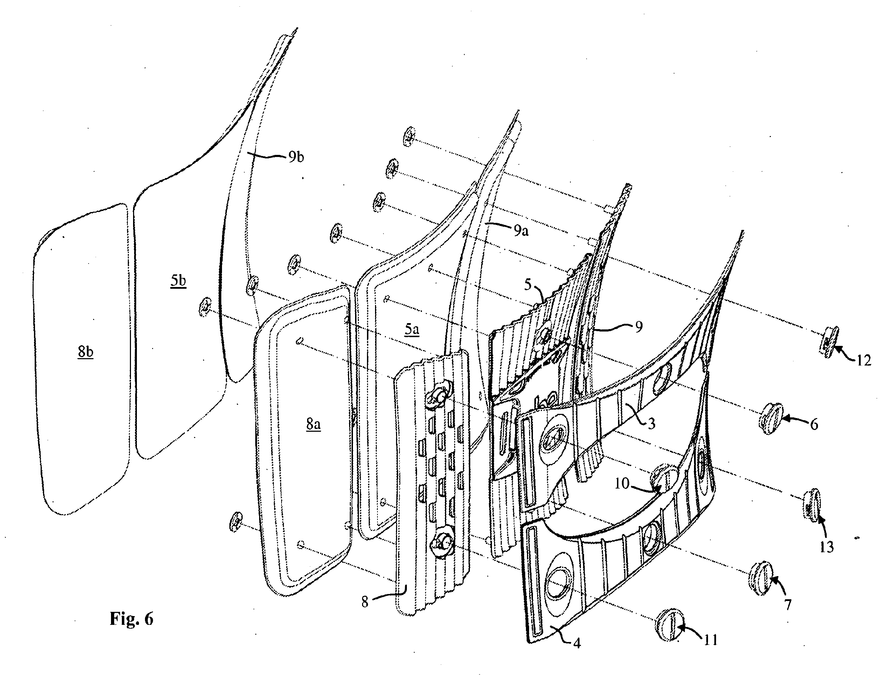

- Figure 6 is an exploded view of the parts forming the lumbar back brace support member according to the present invention.

With reference to the above-cited Figures, the reference numeral 1 indicates a back brace support belt comprising a lumbar back brace support member 2 according to the present invention; the lumbar back brace support member 2 comprises at least a first crosspiece 3 and a second crosspiece 4, which extend on mutually opposite sides along a first axis indicated at A-A, and to which there is pivotally linked at least a backing plate 5 extending along a second axis indicated at B-B that is substantially orthogonal to the first axis A-A, so that, under use conditions, the crosspieces 3 and 4 are oriented in a direction that is substantially transversal relative to the back of the wearer, and the backing plate 5 is arranged at the lumbar region of the same wearer and substantially oriented along the axis of the wearer's spine, corresponding substantially to the second axis B-B. The assembly formed of the crosspieces 3 and 4 and the backing plate 5 is therefore movably jointed in an articulated manner so as to enable the backing plate 5 to pivotally swing relative to the two crosspieces 3, 4, in such a way to follow the movements of the wearer's back, as it will be explained in greater detail further on.

The above-mentioned articulated joint can be obtained in a variety of manners, which may include either a pivotable link between the respective ends of the first crosspiece 3 and the second crosspiece 4, as well as solutions wherein the above-mentioned crosspieces 3, 4 are not mutually linked.

According to a preferred embodiment, illustrated in the accompanying drawings, the lumbar back brace support member 2 comprises a first crosspiece 3 and a second crosspiece 4 that extend on mutually opposite sides spaced from each other relative to the first axis A-A; a first backing plate 5 is pivotally linked to respective central zones of the first crosspiece 3 and the second crosspiece 4 by appropriate first articulated-joint means 6, 7, such as a pair of pins, which are preferably removable so as to allow the backing plate 5 to be possibly replaced if worn out or if the need arises for it to be replaced with another one having a different shape and/or hardness.

In addition, a second backing plate 8 and a third backing plate 9, provided on opposite sides relative to the first backing plate 5, are also pivotally linked to the first crosspiece 3 and the second crosspiece 4 at the respective opposite end portions thereof; appropriate second articulated-joint means 10, 11, such as a pair of pins, which are also preferably removable for the same reasons as set forth above, are provided to pivotally hinge the second backing plate 8 to a respective end portion of the first crosspiece 3 and the second crosspiece 4, whereas similar third articulated-joint means 12, 13 are provided to pivotally hinge the third backing plate 9 to the respective opposite end portion of the first and the second crosspiece 3, 4.

As this can be readily appreciated, one or more such backing plates 5, 8, 9 may of course be associated - at least on the face thereof that comes in contact with the back of the wearer - with appropriate cushioning pads 5a, 8a, 9a removably or fixedly attached to the respective plate. Advantageously, such cushioning pads may be provided through an appropriate vacuum-forming or thermoforming process in view of providing the wearer with an improved anatomic form-fitting capability of the backing plates, such process being in particular adapted to enable cushioning pads to be formed featuring a differentiated pattern of hardness zones so as to provide the wearer with an optimum back support effect accompanied by an improved comfort. The cushioning pads 5a, 8a, 9a may of course be covered with respective sheaths 5b, 8b, 9b.

The way in which the lumbar back brace support member according to the present invention works is as follows: once the wearer has put on the belt, he/she has to position the support member 2 at the lumbar region of the back, so that the first backing plate 5 comes to lie centrally along the axis of the lumbar spine (corresponding to the aforementioned axis B-B) and the second and third backing plates 8, 9 - if used - come to lie laterally, roughly at the renal regions. The articulated construction of the lumbar back brace support member 2 enables the first backing plate 5 and - if used - the second and third backing plates 8, 9 to follow each and any lateral bending movement of the wearer's back through the pivotally swinging movement of the backing plate 5 and - if used - the second and third backing plates 8, 9 relative to the crosspieces 3, 4. In this way, the lumbar back brace support member 2 is capable of following and sustaining the movement of the wearer's back by suitably conforming to the instant position taken by the wearer so as to ensure an optimum support to the lumbar region under any condition of use, while at the same time allowing for an adequate freedom of movement. Furthermore, for each and any position taken by the wearer, the first backing plate 5 is substantially constantly aligned axially with the spine, so as to avoid giving rise to painful points of pressure on the back due to misalignments between the spine and the backing plate.

Fully apparent from the above description is therefore the ability of the lumbar back brace support member according to the present invention to effectively reach the aims and advantages cited afore. As a matter of fact, the lumbar back brace support member for a back brace support belt according to the present invention is able to support and protect the wearer's back in an optimum manner, allowing at the same time for a fully adequate freedom of movement of the same wearer.

Fully apparent is also the ability of a back brace support belt comprising a lumbar back brace support member according to the present invention to ensure - whether it is used in the motorcycling or in the medical field of application - an optimum dynamic support of the lumbar region in each and any position taken by the wearer when making any possible movement, owing to the capability of the backing plates 5, 8, 9 to immediately conform dynamically to the instant position taken by the wearer thanks to the swinging ability thereof.

It should be additionally noticed how the lumbar back brace support member according to the present invention is capable of ensuring a maximum extent of reliability and safety, making it particularly suitable for use in the motorcycling field of application; in fact, the lumbar back brace support member 2 enables the wearer to be provided with an optimum lumbar support effect, while at the same time allowing him/her to perform all lateral movements as needed to ride and control the motorcycle when turning, without this causing him/her to feel painful points of pressure on the back. A further function performed by the lumbar back brace support member according to the present invention is to protect the wearer's back in the event of accidental falls, thanks to the relatively rigid structure of the individual parts forming the lumbar back brace support member.

It shall be appreciated that the materials used, as well as the shape and the sizing of the various parts, may each time be selected so as to more appropriately meet the particular requirements or suit the particular application.

It shall further be appreciated that the various parts forming the object of the present invention shall certainly not be solely embodied in the manner that has been described and illustrated hereinbefore, but can rather be implemented in many other embodiments - although not specifically illustrated here - without departing from the scope defined by the appended claim 1.

## Claims

1. Lumbar back brace support member for a back brace support belt comprising at least a first crosspiece (3) and a second crosspiece (4) substantially extending on opposing and mutually spaced sides relative to a first axis (A-A), and at least a first backing plate (5) extending along a second axis (B-B) in a direction substantially orthogonal to said first axis (A-A), said first backing plate (5) being pivotally linked with said first crosspiece (3) and second crosspiece (4) on opposite sides thereof to respective central zones of said first crosspiece (3) and said second crosspiece (4) by preferably removable first articulated-joint means (6, 7), said first backing plate (5) being substantially oriented, in use, along the axis of the lumbar spine of the wearer, corresponding substantially to said second axis B-B, **characterized in that** said lumbar back brace support member further comprises at least a second backing plate (8) and a third backing plate (9) provided on opposite sides relative to said first backing plate (5), said second backing plate (8) and third backing plate (9) being pivotally linked to the respective opposite end portions of said first crosspiece (3) and said second crosspiece (4) by preferably removable second articulated-joint means (10, 11) and preferably removable third articulated-joint means (12, 13), respectively.

2. Lumbar back brace support member according to claim 1, wherein, under use conditions, said first crosspiece (3) and second crosspiece (4) are oriented in a substantially transverse direction relative to the back of the wearer, and said first backing plate (5), second backing plate (8) and third backing plate (9) are arranged at the lumbar region of said wearer and substantially oriented along the axis of the wearer's spine.

3. Lumbar back brace support member according to any of the preceding claims, wherein, in use, said first backing plate (5), second backing plate (8) and third backing plate (9) follow each and any lateral bending movement of the wearer's back through the pivotally swinging movement of said first backing plate (5), second backing plate (8) and third backing plate (9) relative to said first crosspiece (3) and said second crosspiece (4).

4. Back brace support belt comprising a lumbar back brace support member (2) according to any of the preceding claims.

## Patentansprüche

1. Lumbal-Rückenstützen-Trageelement (2) für einen Rückenstützen-Tragegurt, das wenigstens eine erste Querstrebe (3) und eine zweite Querstrebe (4), die sich im Wesentlichen an einander gegenüberliegenden und voneinander beabstandeten Seiten relativ zu einer ersten Achse (A-A) erstrecken, und wenigstens eine erste Verstärkungsplatte (5) umfasst, die sich entlang einer zweiten Achse (B-B) in einer Richtung im Wesentlichen senkrecht zu der ersten Achse (A-A) erstreckt, wobei die erste Verstärkungsplatte (5) über jeweilige mittige Bereiche der ersten Querstrebe (3) und der zweiten Querstrebe (4) mittels vorzugsweise abnehmbarer erster Drehgelenkeinrichtungen (6, 7) schwenkbar mit der ersten Querstrebe (3) und der zweiten Querstrebe (4) an einander gegenüberliegenden Seiten derselben verbunden ist, und die erste Verstärkungsplatte (5) in Funktion im Wesentlichen entlang der Achse der Lendenwirbelsäule des Trägers im Wesentlichen der zweiten Achse (B-B) entsprechend ausgerichtet ist,
**dadurch gekennzeichnet, dass**
das Lumbal-Rückenstützen-Trageelement (2) des Weiteren wenigstens eine zweite Verstärkungsplatte (8) und eine dritte Verstärkungsplatte (9) umfasst, die an einander gegenüberliegenden Seiten relativ zu der ersten Verstärkungsplatte (5) vorhanden sind, wobei die zweite Verstärkungsplatte (8) und die dritte Verstärkungsplatte (9) mittels vorzugsweise abnehmbarer zweiter Drehgelenkeinrichtungen (10, 11) bzw. vorzugsweise abnehmbarer dritter Drehgelenkeinrichtungen (12, 13) mit jeweiligen einander gegenüberliegenden Endabschnitten der ersten Querstrebe (3) und der zweiten Querstrebe (4) schwenkbar verbunden sind.

2. Lumbal-Rückenstützen-Trageelement (2) nach Anspruch 1, wobei unter Einsatzbedingungen die erste Querstrebe (3) und die zweite Querstrebe (4) in einer im Wesentlichen quer verlaufende Richtung relativ zu dem Rücken des Trägers ausgerichtet sind und die erste Verstärkungsplatte (5), die zweite Verstärkungsplatte (8) und die dritte Verstärkungsplatte (9) an dem Lumbalbereich des Trägers und im Wesentlichen entlang der Achse der Wirbelsäule des Trägers ausgerichtet sind.

3. Lumbal-Rückenstützen-Trageelement (2) nach einem der vorangehenden Ansprüche, wobei im Einsatz die erste Verstärkungsplatte (5), die zweite Verstärkungsplatte (8) und die dritte Verstärkungsplatte (9) jeder etwaigen seitlichen Biegebewegung des Rückens des Trägers über die Schwenkbewegungen der ersten Verstärkungsplatte (5), der zweiten Verstärkungsplatte (8) sowie der dritten Verstärkungsplatte (9) relativ zu der ersten Querstrebe (3) und der zweiten Querstrebe (4) folgen.

4. Rückenstützen-Tragegurt, der ein Lumbal-Rückenstützen-Trageelement (2) nach einem der vorangehenden Ansprüche umfasst.

## Revendications

1. Elément de support d'armatures lombaires pour ceinture orthopédique, comprenant au moins une première pièce transversale (3) et une deuxième pièce transversale (4) s'étendant sensiblement sur des côtés opposés espacés l'un de l'autre par rapport à un premier axe (A-A) et au moins une première plaque de soutien (5) qui s'étend suivant un second axe (B-B) dans une direction sensiblement orthogonale à ladite première direction (A-A), ladite première plaque de soutien (5) étant reliée à pivotement, avec lesdites première pièce transversale (3) et deuxième pièce transversale (5) sur les côtés opposés de celle-ci, à des zones centrales respectives de ladite première pièce transversale (3) et de ladite deuxième pièce transversale (4) par des premiers moyens articulés (6, 7), de préférence amovibles, ladite première plaque de soutien (5) étant sensiblement orientée, en utilisation, suivant l'axe de la partie lombaire de la colonne vertébrale du porteur, correspondant sensiblement au second axe B-B, **caractérisé en ce que** ledit élément de support d'armatures lombaires comprend en outre au moins une deuxième plaque de soutien (8) et une troisième plaque de soutien (9) prévues sur des côtés opposés par rapport à ladite première plaque de soutien (5), lesdites deuxième plaque de soutien (8) et troisième plaque de soutien (9) étant reliées à picotement aux portions d' extrémité opposées respectives de ladite première pièce transversale (3) et ladite deuxième pièce transversale (4) par des deuxièmes moyens articulés (10, 11) de préférence amovibles et par des troisièmes moyens articulés (12, 13) de préférence amovibles, respectivement.

2. Élément de support d'armatures lombaires selon la revendication 1, dans lequel, dans les conditions d'utilisation, lesdites première pièce transversale (3) et deuxième pièce transversale (4) sont orientées dans une direction sensiblement transversale par rapport au dos du porteur, lesdites première plaque de soutien (5), deuxième plaque de soutien (8) et troisième plaque de soutien (9) sont disposées dans la région lombaire dudit porteur et sensiblement orientées suivant l'axe de la colonne vertébrale du porteur.

3. Élément de support d'armatures lombaires selon l'une quelconque des revendications précédentes, dans lequel, en utilisation, lesdites première plaque de soutien (5), deuxième plaque de soutien (8) et troisième plaque de soutien (9) suivent chacune tout mouvement de flexion latérale du dos du porteur grâce au mouvement de pivotement desdites première plaque de soutien (5), deuxième plaque de soutien (8) et troisième plaque de soutien (9) par rapport à ladite première pièce transversale (3) et ladite deuxième pièce transversale (4).

4. Ceinture orthopédique comprenant un élément de support d'armatures lombaires (2) selon l'une quelconque des revendications précédentes.
